# EUROPÄISCHE PATENTSCHRIFT

(11) **EP 0 690 826 B1**
(45) Veröffentlichungstag und Bekanntmachung des Hinweises auf die Patenterteilung: **11.02.1998**
(21) Anmeldenummer: 94912538.9
(22) Anmeldetag: 24.03.1994
(51) Int. Cl.: C02F 3/10, B29C 43/00, B29C 43/22

(54) **FESTBETT-BIOREAKTOR UND TRAGERKÖRPER ZUR REINIGUNG VON FLUIDEN**
FIXED-BED BIOREACTOR AND CARRIER BODIES FOR PURIFYING FLUIDS
BIOREACTEUR A LIT FIXE ET SUBSTRAT D'EPURATION DE FLUIDES

(30) Priorität: 25.03.1993 DE 4309779
(43) Veröffentlichungstag der Anmeldung: 10.01.1996
(73) Patentinhaber: HERDING GMBH, D-92205 Amberg (DE)
(72) Erfinder: HERDING, Walter, D-92256 Hahnbach (DE); VOGEL, Peter, D-92256 Ursula Poppenricht (DE); RABENSTEIN, Klaus, D-92256 Hahnbach-Süss (DE)
(74) Vertreter: Klunker . Schmitt-Nilson . Hirsch
(86) Internationale Anmeldenummer: EP9400945
(87) Internationale Veröffentlichungsnummer: WO9421566

(56) Entgegenhaltungen:
- EP-A- 0 137 449
- EP-A- 0 332 907
- DE-A- 3 149 298
- DE-A- 4 107 406
- US-A- 2 458 163
- US-A- 3 231 490

## Beschreibung

Die Erfindung bezieht sich auf einen Festbett-Bioreaktor zur Reinigung Von Fluiden mit Hilfe von Mikroorganismen, der eine Mehrzahl von Trägerkörpern für Mikroorganismen und Strömungswege für das Fluid entlang der Trägerkörper enthält, wobei der Trägerkörper flächige Gebilde mit im Vergleich zu der Trägerkörperfläche Kleiner Dicke sind, einen porösen Aufbau mit von dem Fluid durchsetzbaren und von Mikroorganismen besiedelbaren Poren haben, und mit durch Wärmeeinwirkung verbundenen Kunststoffteilchen aufgebaut sind. Bei einem bekannten Festbett-Bioreaktor dieser Art (WO92/22505) sind die Trägerkörper ebene Platten oder kreisförmig gerundete Platten. Die Trägerkörper werden jeweils an ihrem oberen und an ihrem unteren Ende durch Halterungen an Ort und Stelle und auf gegenseitigen Abstand gehalten.

Der Erfindung liegt die Aufgabe zugrunde, einen Festbett-Bioreaktor verfügbar zu machen, dessen Trägerkörper unkompliziert und rationell herstellbar sind und besonders einfach in sinnvoller Relativanordnung zueinander in dem Bioreaktor positioniert werden können.

Zur Lösung dieser Aufgabe ist der Festbett-Bioreaktor erfindungsgemäß dadurch gekennzeichnet, daß die Trägerkörper mit Hilfe von Distanzstegen voneinander beabstandet sind, wodurch die Strömungswege zwischen den Trägerkörpern definiert sind; und daß die Distanzstege integral mit dem Kunststoffmaterial eines jeweils zugeordneten Trägerkörpers angeformt sind.

Festbett-Bioreaktoren mit Trägerkörpern, die mit Hilfe von Distanzelementen voneinander beabstandet sind, sind aus US-A-2 458 163 und DE-A-41 07 406 bekannt. Dort haben die Trägerkörper jedoch keinen porösen Aufbau aus durch Wärmeeinwirkung verbundenen Kunststoffteilchen; die Distanzstege sind nicht integral angeformt.

Die einfachste Form eines geeigneten, flächigen Gebildes ist die Plattenform; im wesentlichen plattenförmige Trägerkörper sind insofern besonders bevorzugt. Aber auch von der exakten, ebenen Plattenform abweichende Konfigurationen sind günstig einsetzbar, insbesondere Konfigurationen nach Art gewellter Platten, wobei der Verlauf relativ zu der Hauptplattenebene mehr gerundet oder mehr geradlinig-abknickend sein kann. Es ist jedoch bevorzugt, wenn auch in den letztgenannten Fallen die Gestalt des jeweiligen Trägerkörpers - insgesamt und grob betrachtet - plattenförmig ist, denn dann lassen sich die Trägerkörper besonders günstig in den Bioreaktor einbauen.

Die Materialdicke der Trägerkörper muß nicht für den gesamten jeweiligen Trägerkörper konstant sein. Es ist jedoch bevorzugt, wenn der jeweilige Trägerkörper überall mindestens im wesentlichen die gleiche Materialdicke hat. Unter "Trägerkörperfläche" wird bei einem streng plattenförmigen Trägerkörper die Größe einer Flachseite des Trägerkörpers verstanden, bei wellplattenförmigen Gebilden die Größe der entsprechenden Flachseite, die man sich als durch Wellung der ebenen Flachseite einer Platte entstanden denken kann.

Die Trägerkörper können an einer Trägerkörperfläche oder an beiden Trägerkörperflächen integral mit Distanzstegen ausgebildete sein. Man kann die Trägerkörper besonders einfach aneinandersetzen, indem die Distanzstege an den oder die benachbarten Trägerkörper anstoßen, und zwar entweder unmittelbar oder an Distanzstege der benachbarten Trägerkörper.

Es ist bevorzugt, mehrere Trägerkörper zu einem Tragerkörper-Paket zu vereinigen und als miteinander verbundenes Paket in den Bioreaktor einzubauen. Dies geschieht insbesondere durch Verbinden der integralen Distanzstege eines betrachteten Trägerkörpers mit dem auf einer Seite benachbarten Trägerkörper oder den auf beiden Seiten benachbarten Tragerkörpern. Für das Verbinden der Distanzstege mit Trägerkörpern oder auch der Distanzstege benachbarter Trägerkörper miteinander sind Klebung oder Schweißung besonders bevorzugt.

Es wird darauf hingewiesen, daß die Distanzstege zwar vorzugsweise die Gestalt länglicher Materialstreifen haben, daß es jedoch für die Funktion der Distanzstege und des Bioreaktors nicht darauf ankommt, daß es sich um kontinuierlich durchgehende Distanzstege pro Trägerkörper handelt. So können z.B. die Distanzstege beabstandete Öffnungen aufweisen oder sich aus einer Reihe von beabstandeten Distanzelementen zusammensetzen. Die Distanzstege können massiv oder porös sein.

Vorzugsweise sind durch Wärmeeinwirkung verbundene Kunststoffteilchen der Hauptbestandteil der Trägerkörper. Polyethylen-Teilchen sind besonders bevorzugt, wobei jedoch auch andere Kunststoffe geeignet sind. Vorzugsweise enthalten die Trägerkörper zusätzlich feinporöse Teilchen, so daß sich ein Gesamtaufbau der Trägerkörper mit größeren Poren z.B. zwischen den Kunststoffteilchen und kleineren Poren innerhalb der feinporösen Teilchen ergibt. Die Mikroorganismen siedeln dann nicht nur in den gröberen Poren, sondern auch in den feineren Poren. Geeignete feinporöse Teilchen bestehen z.B. aus geschrotetem Blähton, Aktivkohle oder anderen organischen Stoffen. Wichtig ist, daß die Trägerkörper insgesamt, also auch die mikroporösen Teilchen, von dem zu reinigenden Fluid durchsetzbar sind. Die Trägerkörper sind also offenporige, gegebenenfalls einschließlich der feinporösen Teilchen, durchströmbare Gebilde.

Die Trägerkörper können außerdem Zusätze enthalten, wobei als Beispiel Nickel, das eine katalytische Wirkung für die Lebensvorgänge der Mikroorganismen hat, erwähnt wird.

Ein weiterer Gegenstand der Erfindung sind Trägerkörper für Festbett-Biorekatoren zur Reinigung von Fluiden mit Hilfe von Mikroorganismen, wobei der Trägerkörper ein flächiges Gebilde mit im Vergleich zu der Trägerkörperfläche kleiner Dicke ist, einen porösen Aufbau mit von dem Fluid durchsetzbaren und von Mikroorganismen besiedelbaren Poren hat, und mit durch Wärmeeinwirkung verbundenen Kurststoffteilchen aufgebaut ist, dadurch gekennzeichnet, daß der Trägerkörper integral mit seinem Kunststoffmaterial angeformte Distanzstege zur Definition des Abstands zu einem benachbarten Trägerkörper aufweist.

Die vorstehend dargelegten Gesichtspunkte und die vorstehend angesprochenen, bevorzugten Ausgestaltungen gelten analog auch für die erfindungsgemäßen Trägerkörper.

Die Erfindung bezieht sich ferner auf ein Verfahren zur Herstellung von zur Besiedlung mit Mikroorganismen geeigneten Trägerkörpern porösen Aufbaus, wobei das Verfahren folgende Schritte aufweist:
(a) Bereitstellen von Kunststoffteilchen;
(b) Einbringen der Kunststoffteilchen in einen Formgebungsraum; und
(c) Wärmezufuhr zu den im Formgebungsraum enthaltenen Kunststoffteilchen, so daß sich die Kunststoffteilchen unter Ausbildung des porösen Trägerkörperaufbaus miteinander verbinden,
   dadurch gekennzeichnet,
(d) daß der Inhalt des Formgebungsraums durch Bewegung bewegbarer Formgebungsraum-Begrenzungen in Richtung zu einem Abgabeende des Formgebungsraums mitgenommen wird und im Verlauf der Mitnahmebewegung durch Wärmezufuhr zu dem porösen Trägerkörperaufbau verbunden wird.

Schließlich bezieht sich die Erfindung auf ein Verfahren zur Herstellung von zur Besiedlung mit Mikroorganismen geeigneten Trägerkörpern porösen Aufbaus, wobei das Verfahren folgende Schritte aufweist:
(a) Bereitstellen von Kunststoffteilchen;
(b) Einbringen der Kunststoffteilchen in einen Formgebungsraum; und
(c) Wärmezufuhr zu den im Formgebungsraum enthaltenen Kurststoffteilchen, so daß sich die Kunststoffteilchen unter Ausbildung des porösen Trägerkörperaufbaus miteinander verbinden,
   dadurch gekennzeichnet,
(d) daß der jeweilige Formgebungsraum von einer ersten und einer zweiten Formhälfte begrenzt ist;
(e) daß eine Reihe der ersten Formhälften an einem ersten Förderer und eine Reihe der zweiten Formhälften an einem zweiten Förderer befestigt ist;
(f) daß die Formhälften in geschlossenem Zustand durch eine Station zum Einbringen der Kunststoffteilchen und durch eine Station zur Wärmezufuhr zu den beinhalteten Kunststoffteilchen bewegbar sind; und
(g) daß die Formhälften aufgrund der Ausbildung des ersten und des zweiten Förderers selbsttätig geöffnet und geschlossen werden.

Die erfindungsgemäßen Herstellungsverfahren können so gefühlt werden, daß sich ein kontinuierlicher oder ein halbkontinuierlicher oder ein fließbandartig-taktweiser Herstellungsgang ergibt.

Wenn man den Formgebungsraum derart gestaltet und verfahrensmäßig betreibt, daß er eine kontinuierliche Strangformgebung leistet, vorzugsweise durch Begrenzen des Formgebungsraums auf zwei gegenüberliegenden Seiten durch endlose, umlaufend bewegte Bänder oder durch rotierbare Walzen ausreichend großen Durchmessers, und wenn man darüberhinaus die Kunststoffteilchen dem Formgebungsraum kontinuierlich oder quasi-kontinuierliche zufährt, verwirklicht man ein kontinuierliches Herstellungsverfahren. Wenn ein endloser Strang von Trägerkörpermaterial aus dem Formgebungsraum abgezogen wird, lassen sich von dem Strang einzelne Trägerkörper abtrennen.

Alternativ kann man den Formgebungsraum durch öffenbare und schließbare Formteile, insbesondere Formhälften, begrenzen und chargenweise, jeweils bei geschlossenen Formteilen, die Kunststoffreilchen einbringen. Diese "stuckweise" Erzeugung schließt nicht aus, daß schrittweise und intermittierend ein endloser Strang von Trägerkörpermaterial gebildet wird. Ein derartiges Herstellungsverfahren kann man als halbkontinuierlich bezeichnen.

Um die für das gegenseitige Verbinden der Kunststoffteilchen unter Wärmeeinwirkung erforderliche Verweilzeit im Formgebungsraum zu verkürzen, kann man die Kunststoffteilchen vor dem Einbringen in den Formgebungsraum erwärmen. Für die Wärmezufuhr zu den im Formgebungsraum enthaltenen Kunststoffteilchen gibt es eine Reihe technischer Möglichkeiten. Als bevorzugte Beispiele seien die Wärmezufuhr durch Erwärmen von Begrenzungsflächen des Formgebungsraums und/oder durch Mikrowellen und/oder durch Wärmestrahlung und/oder durch die Zugabe vorgewärmter, feinporöser Teilchen.

Die erfindungsgemäßen Herstellungsverfahren, auch die kontinuierlich arbeitenden; gestatten bei entsprechender Gestaltung von Formgebungsraumbegrenzungen die besonders rationelle Herstellung von Trägerkörpern mit einstückig angeformten Distanzstegen. Nach einer besonders bevorzugten Weiterbildung der erfindungsgemäßen Verfahren sollen Trägerkörper mit Distanzstegen unterschiedlicher Höhe hergestellt werden können, da je nach zu reinigendem Fluid und je nach für die Reinigung eingesetzten Mikroorganismen Strömungswege unterschiedlicher Abmessung von Trägerkörper zu Trägerkörper günstig sind. Zu diesem Zweck kann man insbesondere mit auswechselbaren Formgebungsraumbegrenzungen arbeiten. Eine andere bevorzugte Alternative besteht darin, eine Formgebungsraumbegrenzung mit einer oder mehreren Nuten (zur Formung der Distanzstege) vorzusehen, wobei die Nuttiefe der maximal herzustellenden Distanzsteghöhe entspricht. Wenn Trägerkörper mit Distanzstegen geringerer Höhe hergestellt werden sollen; kann man in den Nuten Materialeinlagen anordnen, welche die effektive Nuttiefe verringern.

Mittels der erfindungsgemäßen Herstellungsverfahren lassen sich auch Trägerkörper herstellen, die mikroporöse Teilchen enthalten, wie weiter vom ausgeführt. Zu diesem Zweck muß man lediglich den Kunststoffteilchen, die in den Formgebungsraum eingebracht werden, derartige mikroporöse Teilchen zusetzen, am besten in gleichmäßig verteilter Form.

Mit Hilfe des erfindungsgemäßen Bioreaktors bzw. der erfindungsgemäßen Trägerkörper laßt sich eine Vielzahl von Fluiden behandeln. Als typische Beispiele seien, insbesondere hochbelastete, Abwässer unterschiedlichster Herkunft (insbesondere organisch belastete Abwässer z.B. von Schlachthöfen, Brauereien, Molkereien, allgemein von Anlagen der Nahrungsmittelindustrie) genannt. Der Bioreaktor ist vorzugsweise ein anaerob arbeitender Bioreaktor, kann aber auch ein anoxisch oder aerob arbeitender Bioreaktor sein. Es können entweder Mikroorganismen mit anabolem Stoffwechselprozeß, d.h. gerichtet auf den Abbau von bestimmten Produkten oder Schadstoffen, eingesetzt werden. Oder es können Mikroorganismen mit katäbolem Stoffwechselprozeß eingesetzt werden, also gerichtet auf den Aufbau bestimmter gewünschter Stoffwechselprodukte, z.B. Erzeugung von Antibiotika, Alkohol u.a.. Die Behandlung von Gasen und Flüssigkeiten kann miteinander kombiniert sein.

Hinsichtlich der erfindungsgemäßen Herstellungsverfahren für Trägerkörper wird ergänzend darauf hingewiesen, daß die erzeugten Trägerkörper bzw. der erzeugter Trägerkörperstrang vor und/oder während und/oder nach dem Ausbringen aus dem Formgebungsraum normalerweise abgekühlt wird, z.B. durch Anblasen mit Luft.

Die Erfindung und Ausgestaltungen der Erfindung werden nachfolgend anhand von schematisiert zeichnerisch dargestellten Ausführungsbeispielen noch näher erläutert. Es zeigt:
- **Fig. 1**: eine Abwasser-Reinigungsanlage mit einem Festbett-Bioreaktor;
- **Fig. 2a**: einen horizontalen Querschnitt des Bioreaktors von **Fig. 1**;
- **Fig. 2b**: einen horizontalen Querschnitt eines abgewandelten Bioreaktors;
- **Fig. 3**: einen Trägerkörper mit integralen Distanzsiegen des Bioreaktors von **Fig. 1** in perspektivischer Darstellung;
- **Fig. 4**: eine Anordnung von zwei Trägerkörpern gemäß **Fig. 3** in einem horizontalen Querschnitt;
- **Fig. 5**: eine abgewandelte Ausführungsform eines Trägerkörpers mit integralen Distanzstegen im horizontalen Querschnitt;
- **Fig. 6**: eine weitere abgewandelte Ausführungsform eines Trägerkörpers mit integralen Distanzstegen im horizontalen Schnitt, und zwar als Anordnung mehrerer Trägerkörper;
- **Fig. 7**: in einem horizontalen Schnitt eine Anordnung mehrerer Trägerkörper mit dazwischen angeordneten, separaten Distanzstegen;
- **Fig. 8**: ein Trägerkörper-Paket aus einer Mehrzahl von Trägerkörpern;
- **Fig. 9**: den Formgebungsraum einer Anlage zur kontinuierlichen Herstellung von Trägerkörpern;
- **Fig. 10**: den Formgebungsraum einer weiteren Ausführungsform einer Anlage zur kontinuierlichen Herstellung von Trägerkörpern;
- **Fig. 11**: den Formgebungsraum einer weiteren Ausführungsform einer Anlage zur kontinuierlichen Herstellung von Trägerkörpern;
- **Fig. 12**: schematisiert eine Anlage zur halbkontinuierlichen Herstellung von Trägerkörpern;
- **Fig. 13**: schematisiert eine Anlage zur taktweisen Herstellung von Trägerkörpern;
- **Fig. 13a**: schematisiert die Anlage von **Fig. 13** in Seitenansicht gemäß Pfeil;
- **Fig. 14**: einen erfindungsgemäßen Trägerkörper im Schnitt.

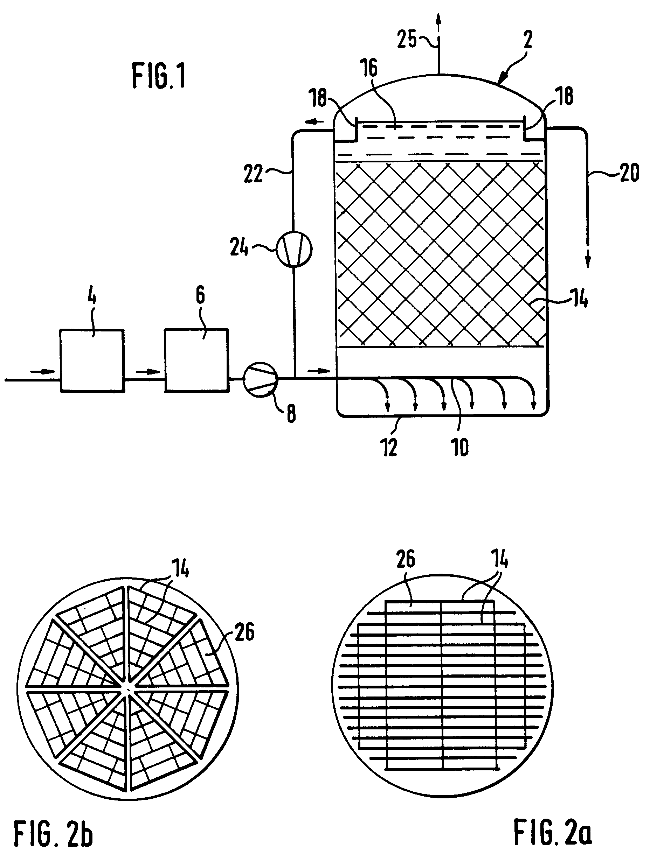

Die in **Fig. 1** mit ihren Hauptbestandteilen gezeigte Anlage zur biologischen Behandlung von Abwasser besteht im wesentlichen aus einem Festbett-Bioreaktor 2, dem ein Behälter 4 mit quasi-kontinuierlichem oder semikontinuierlichem Durchlauf zur Neutralisation des zu behandelnden Abwassers und ein Wärmetauscher 6 vorgeschaltet sind, welcher das zu behandelnde Abwasser auf eine für die biologische Behandlung günstige Temperatur bringt. Mittels einer Pumpe 8 und eines Rohrverteilungssystems 10 wird das Abwasser gleichmäßig über den Boden 12 des Bioreaktors 2 verteilt mit nach unten gerichteten Strahlen im unteren Bereich des Bioreaktors 2 in diesen eingepumpt. Der Bioreaktor 2 hat insgesamt die Konfiguration eines aufrecht stehenden Zylinders, wobei die obere Abschlußwand etwas nach oben gewölbt sein kann. Alternativ kann der Bioreaktor 2 einen quadratischen oder einen rechteckigen Querschnitt haben. Bevorzugte Materialien für den Bioreaktor 2 sind Edelstahl und, insbesondere bei sehr großem Volumen, Beton.

Oberhalb des Rohrverteilungssystems 10 ist in dem Bioreaktor 2 eine Vielzahl von Trägerkörpern 14 angeordnet, wie weiter unten noch ausführlicher beschrieben wird. Oberhalb der Trägerkörper 14 befindet sich in dem Bioreaktor 2 eine Beruhigungszone 16 mit Schlammabsetzraum.

Hinter einem Überlaufwehr 18 geht eine Ablauf-Rohrleitung 20 von dem Bioreaktor 2 ab. Außerdem erkennt man eine Rezirkulationsleitung 22 mit einer darin eingebauten Pumpe 24. Durch die Rezirkulationsleitung kann Abwasser aus der Beruhigungszone 16 abgezogen und dem Rohrverteilungssystem 10 zugeführt werden. Mittels der Rezirkulation eines Teils des Abwassers, welches die Anordnung der Trägerkörper 14 bereits passiert hat, läßt sich die mittlere Verweilzeit des Abwassers im Bioreaktor steigern. Durch eine zentrale obere Rohrleitung 25 kann gebildetes Gas abströmen.

In **Fig. 2a** erkennt man, daß die Trägerkörper 14 die Konfiguration ebener, aufrecht gestellter Platten haben. Die Trägerkörper sind parallel zueinander angeordnet und haben - waagerecht gemessen - eine unterschiedliche Breite, damit der Innenraum des Bioreaktors 2 möglichst vollständig genutzt wird. Die Abwasserströmung im Bioreaktor 2 ist von unten nach oben entlang der Flachseiten der plattenförmigen Trägerkörper 14, und zwar durch die Abstandsräume 26 jeweils zwischen zwei benachbarten Trägerkörpern 14. Alternativ könnte man einen Bioreaktor mit Abwasserströmung von oben nach unten bauen. Die Trägerkörper 14 müssen nicht so breit sein wie der Bioreaktor 2, sondern könnten jeweils in mehrere Trägerkörper nebeneinander unterteilt sein.

Bei der Variante gemaß **Fig. 2b** bilden jeweils mehrere parallele Trägerkörper 14 ein im Querschnitt dreieckiges Trägerkörperpaket. Mehrere Trägerkörperpakete sind umfangmäßig aneinandergesetzt, so daß sich insgesamt eine Konfiguration mit im Querschnitt polygonalem, nahezu kreisförmigem Außenumfang ergibt, wobei die Trägerkörper 14 jeweils tangential verlaufen.

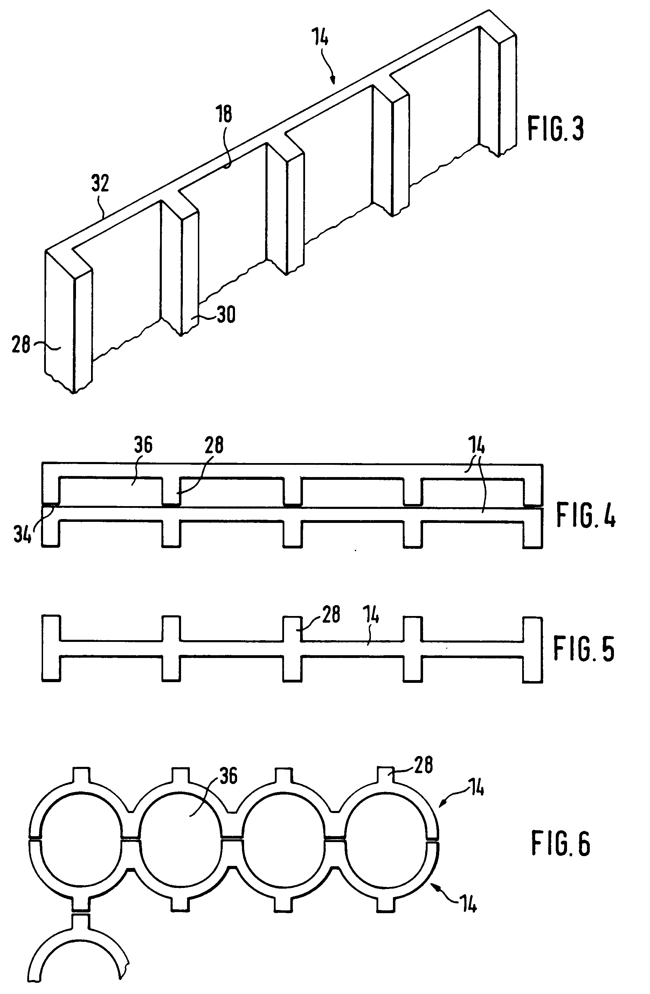

In **Fig. 3** erkennt man den Aufbau eines Trägerkörpers mehr im einzelnen. An einer Flachseite 18 des Trägerkörpers 14 sind integral angeformte, vorstehende Distanzstege 28 vorgesehen. Die Distanzstege 28 verlaufen, wenn der Trägerkörper 14 in den Bioreaktor 2 eingebaut ist, vertikal. Die Endflächen 30 aller Distanzstege 28 liegen in einer gemeinsamen Ebene. An der gegenüberliegenden Flachseite 32 weist der Trägerkörper 14 keine Distanzstege 28 auf, sondern ist durchgehend eben.

**Fig. 4** veranschaulicht, wie man die Trägerkörper von **Fig. 3** parallel aneinandersetzen kann, um eine Anordnung einer Vielzahl von Trägerelementen 14 zu schaffen. Man setzt die Endflächen 30 der Distanzstege 28 des in **Fig. 4** oberen, ersten Trägerkörpers gegen die Flachseite 32 des in **Fig. 4** unteren, zweiten Trägerkörpers 14 und stellt an den sich so ergebenden Berührungsflächen 34 eine feste Verbindung zwischen den zwei benachbarten Trägerkörpern 14 her, vorzugsweise durch Verklebung oder Verschweißung. Die Anordnung wird fortschreitend nach unten in **Fig. 4** analog fortgesetzt, bis ein Paket aus z.B. zehn bis 100 miteinander verbundenen Trägerkörpern 14 gebildet ist.

Es versteht sich, daß die in **Fig. 4** horizontal gemessene Breite der Trägerkörper 14 variieren kann, um die Gesamtgestalt des Trägerpakets z.B. einem zylindrischen Bioreaktor 2 anzupassen, wie in **Fig. 2** gezeigt. Man kann mehrere der Trägerpakete gemäß **Fig. 4** nebeneinander in dem Bioreaktor 2 anordnen. Es versteht sich ferner, daß diese Länge der Trägerkörper 14 je nach Höhe des Bioreaktors 2 passend gewählt ist.

Jeweils an zwei gegenüberliegenden Seiten begrenzt durch Distanzstege und jeweils an den zwei anderen gegenüberliegenden Seiten begrenzt durch Trägerkörper-Flächen 18 bzw. 32 sind Strömungswege 36 definiert, die im wesentlichen einen rechteckigen Querschnitt haben. Es wird darauf hingewiesen, daß die Distanzstege 28 nicht notwendigerweise eine durchgehende, leistenförmige Gestalt haben müssen, wie in **Fig. 3** gezeichnet. Gewisse Querströmungen von Strömungsweg 36 zu Strömungsweg 36 sind nicht störend. Funktionell kommt es in erster Linie darauf an, die Trägerkörper 14 mittels der Distanzstege 28 stabil und beabstandet zu positionieren.

In **Fig. 5** ist eine Variante gezeichnet, bei der die Trägerkörper 14 auf ihren beiden Flachseiten jeweils mit integralen Distanzstegen 28 versehen sind. Beim Ansetzen eines benachbarten Trägerkörpers 14 kommt jeweils Distanzsteg 28 gegen Distanzsteg 28.

**Fig. 6** zeigt ein weiteres Beispiel aus einer Vielzahl möglicher Varianten für die Geometrie der Trägerkörper 14. Gezeichnet ist eine Variante, die man als gewellte Platte bezeichnen kann, wobei gleichsam etwa halbkreisförmige Wölbungen in Reihe aufeinander folgen. Auch hier erkennt man wiederum an den beiden "Großflächenseiten" der Trägerkörper 14 integral angeformte Distanzstege 28. Benachbarte Trägerkörper 14 sind jeweils spiegelbildlich aneinandergesetzt. Es ergeben sich Strömungswege 36 in zwei Geometrien, nämlich im wesentlichen kreisförmig und im wesentlichen quadratisch mit nach innen eingebuchteten Begrenzungswänden.

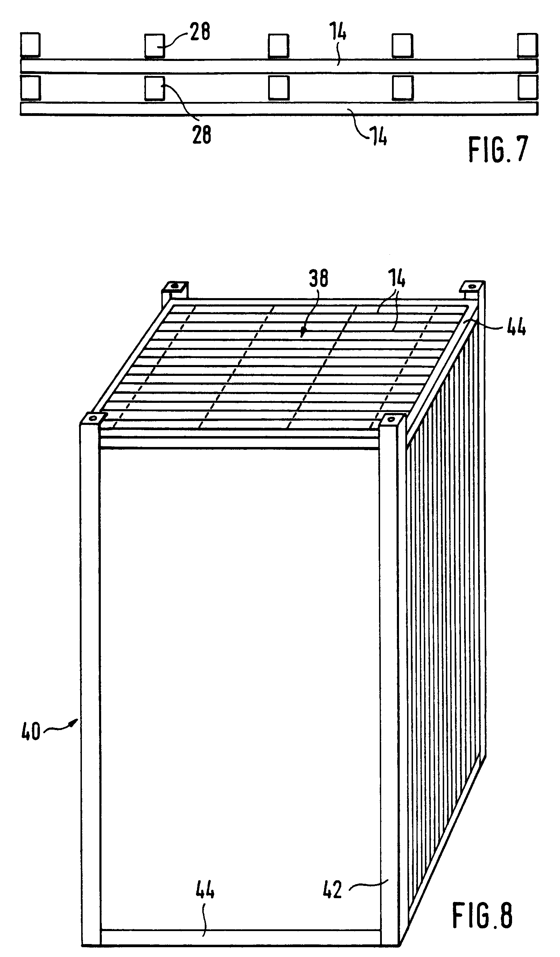

In **Fig. 7** ist eine Variante veranschaulicht, bei der die Distanzstege 28 nicht integral an die plattenförmigen Trägerkörper 14 angeformt sind, sondern in Form separater streifenförmiger Elemente vorgesehen sind. Die Distanzstege 28 sind jeweils mit den beiden benachbarten Trägerkörpern 14 verbunden, zum Beispiel durch Verklebung oder Verschweißung, so daß insgesamt ein Trägerkörperpaket analog zur Ausführungsform gemäß **Fig. 4** gebildet ist.

**Fig. 8** veranschaulicht, wie ein Trägerpaket 38 in einer Art Rahmen 40 angebracht ist. Der Rahmen 40 besteht im wesentlichen aus vier vertikalen Winkelprofilen 42 an den Ecken des Trägerkörperpakets 38. Diese Winkelprofile 42 sind oben und unten durch jeweils vier Verbindungsstäbe 44 miteinander verbunden. In dieser Gesamtanordnung wird das Trägerkörperpaket 38 in den Bioreaktor 2 eingebaut, wo z.B. der Rahmen 40 auf dem Boden des Bioreaktors 2 aufruht Bei relativ hohen Bioreaktoren 2 kann man mehrere derartige Trägerkörperpakete 38 übereinander anordnen. Eine typische Höhe des Trägerkörperpakets 38 ist zwischen 1 und 2 m. Die horizontalen Abmessungen können zum Beispiel 0,5 bis 2 m² sein.

Die Trägerkörper 14 bestehen vorzugsweise aus durch Wärmeeinwirkung miteinander verbundenen Kunststoffteilchen, z.B. aus Teilchen aus Polyethylen mittlerer Dichte. Die Kunststoffteilchen haben typischerweise eine Teilchengröße, die hauptsächlich im Bereich von 200 bis 3000 µm ist, wobei der Weit überwiegende Teil der Teilchen eine Teilchengröße von 630 bis 1600 µm hat. Außerdem enthält der Trägerkörper 14 geschrotete Blähton-Teilchen, die in sich mikroporös sind. Die Größe der Poren zwischen den Kunststoffteilchen beträgt 0,1 bis 5000 µm, wobei der weit überwiegende Teil der Poren im Porengrößenbereich von 100 bis 500 µm liegt. Die Poren in den Blähton-Teilchen sind im Mittel deutlich kleiner.

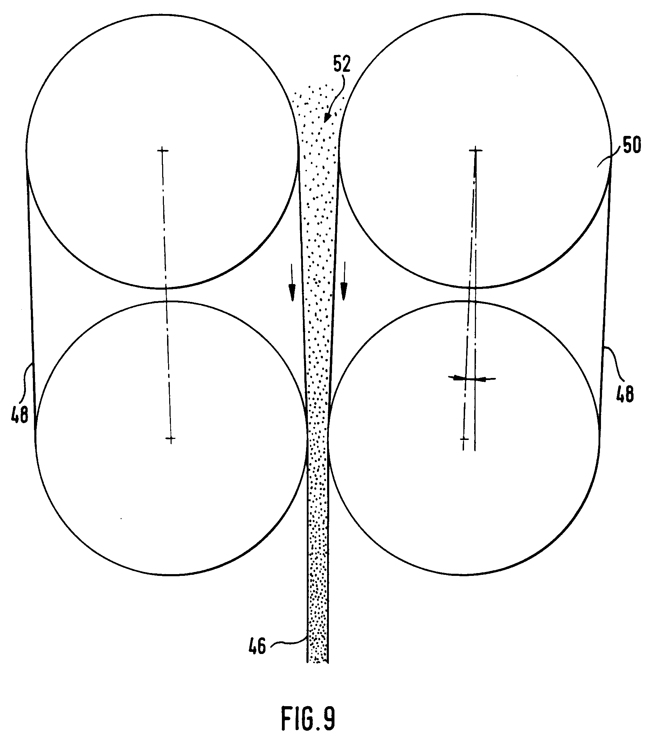

In **Fig. 9** ist gezeichnet, wie man einen Strang 46 aus porösem Trägerkörpermaterial kontinuierlich herstellen kann. Es sind zwei endlose Metallbänder 48 vorgesehen die jeweils um zwei beabstandete Walzen 50 geführt sind. Im Zentrum der Anordnung verlaufen die beiden Metallbänder 48 nahezu parallel zueinander, jedoch unten in **Fig. 9** zwischen den beiden unteren Walzen mit etwas kleinerem Abstand als oben in **Fig. 9** zwischen den beiden oberen Walzen. Von oben wird in den - grob gesprochen - trichterförmigen Raum 52 zwischen den beiden Bändern 48, die dort um die beiden oberen Walzen 50 herumlaufen, das Rohmaterial, nämlich Kunststoffteilchen und geschrotete Blähton-Teilchen, eingebracht. Die Bänder 48 sind beheizt, und die Länge des Kontakts des Kunststoffmaterials mit den beiden Bändern 48 ist so bemessen und mit der Bewegungsgeschwindigkeit der Bänder 48 abgestimmt, daß die Zeit ausreicht, um die Kunststoffteilchen durch Temperaturerhöhung an ihrer Oberfläche klebrig zu machen und zum gegenseitigen Verbinden zu bringen. Hierfür ist ein Temperaturbereich von 160 bis 190°C geeignet. Beim Austritt aus dem Paar der Bänder 48 wird der Strang gekühlt. Weiter hinten wird der Strang in die einzelnen Trägerkörper 14 quer zu seiner Längsrichtung zerschnitten.

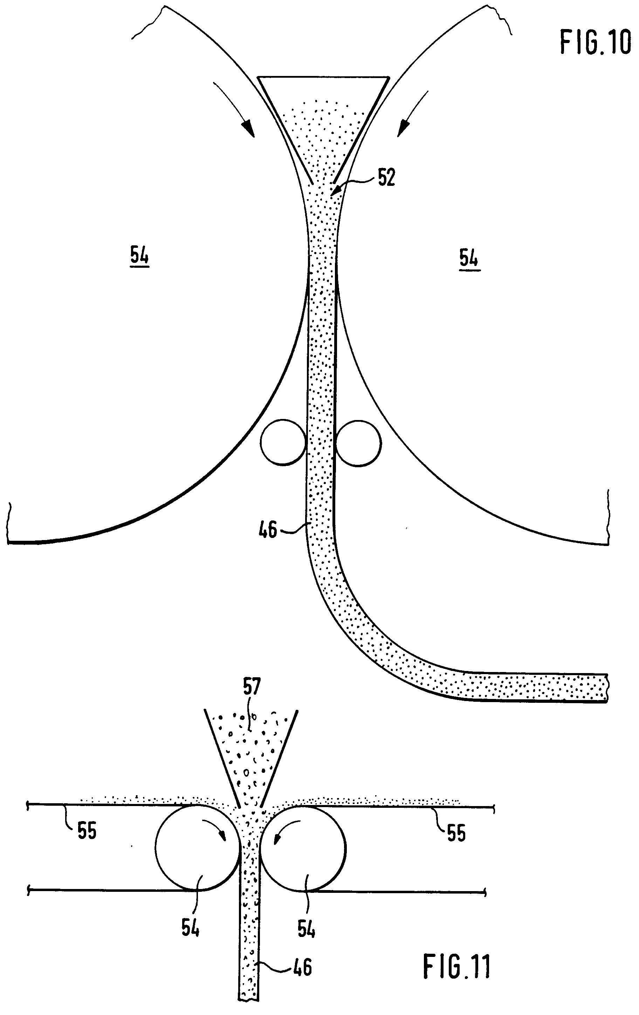

**Fig. 10** zeigt eine Variante`, bei der die beiden Bänder 48 durch ein Paar von Walzen 54 ersetzt sind. Der Verfahrensablauf ist grundsätzlich der gleiche, wie vorstehend geschildert.

**Fig. 11** zeigt eine der Ausführungsform gemäß **Fig. 10** ähnliche Variante, bei der jedoch jede der beiden Walzen 54 End-Umlenkwalze eines horizontalen endlosen Förderbands 55 aus Metall ist. Die Kunststoffteilchen werden auf den Förderbändern 55 liegend dem Formgebungsraum zwischen den beiden Walzen 54 zugefördert. Von oben fallen aus einem Vorratstrichter 57 geschrotete Blähton-Teilchen in den "Formgebungsspalt". Die Blähton-Teilchen sind auf z.B. über 200°C vorgewärmt und bringen soviel Wärme in den Formgebungsraum ein, daß dort das gegenseitige Verbinden der Kunststoffteilchen unter Inkorporieren der Blähton-Teilchen stattfindet.

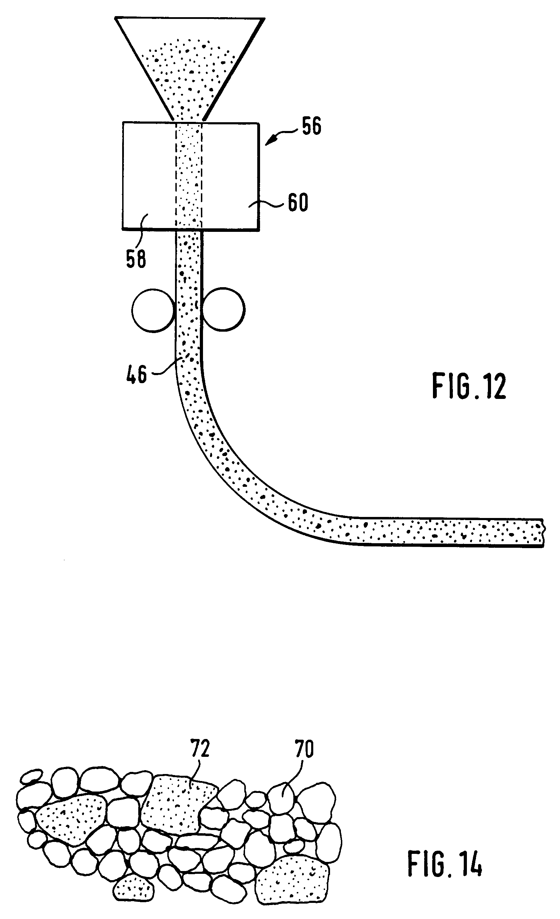

**Fig. 12** veranschaulicht ein Verfahren, welches hinsichtlich der Formgebung des Strangs 46 als Schrittabfolge abläuft. Statt des Paars von Bändern 48 bzw. von Walzen 54 ist eine Form 56 aus zwei Formhälften 58 und 60 vorgesehen. Bei geschlossenen Formhälften 58, 60 wird von oben in den Formhohlraum das Rohmaterial eingebracht. Nach dem Einbringen können die Formhälften 58, 60 noch ein Stück weiter aufeinander zu bewegt werden, um Druck auf den Forminhalt auszuüben. Zu dieser Zeit steht der herzustellende Strang 46 still. Dem Forminhalt wird Wärme zugeführt zur gegenseitigen Verbindung der Kunststoffteilchen. Dann werden die Formhälften 58, 60 leicht geöffnet und wird der herzustellende Strang 46 uni eine Länge, die im wesentlichen der Höhe der Form 56 entspricht, nach unten abgezogen. Die Form 56 wird wieder geschlossen, und der Vorgang wiederholt sich.

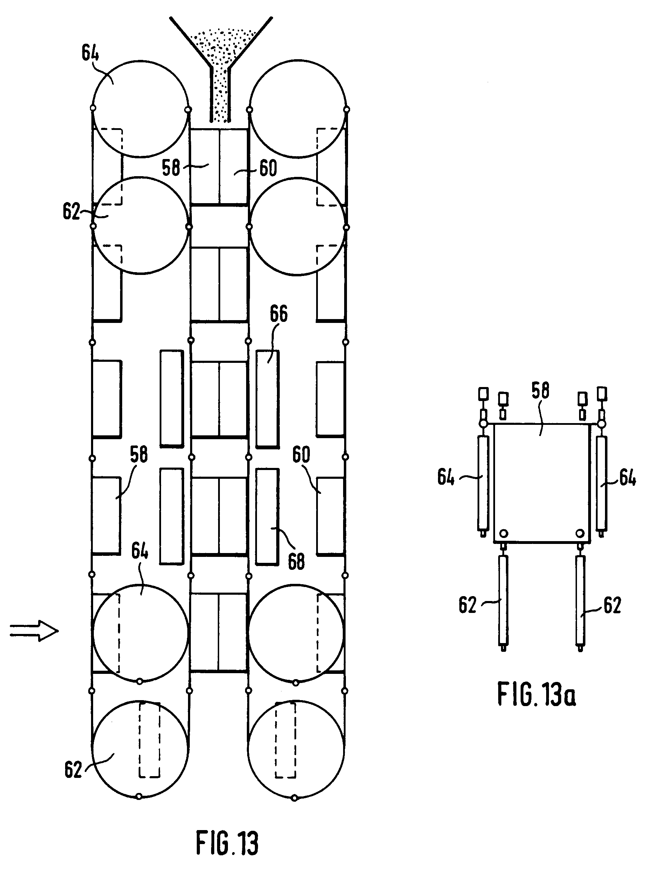

In **Fig. 13** ist ein Herstellungsverfahren veranschaulicht, welches nicht zu einem kontinuierlichen Strang 46 aus Trägerkörpermaterial führt sondern taktweise zu einer Folge einzelner Trägerkörper 14. Linke Formhälften 58 analog der linken Formhälfte 58 von **Fig. 12** sind in Reihe an zwei Förderern, jeweils in Form eines Paars endloser Förderketten, befestigt. Die unteren Enden der in **Fig. 13** linken Formhälften sind an einem Paar von Förderketten befestigt, die über untere Kettenräder 62 umgelenkt werden, während die in **Fig. 13** oberen Enden der in **Fig. 13** linken Formhälften an einem weiteren Kettenpaar befestigt sind, dessen Ketten durch obere Umlenkräder 64 umgelenkt werden. Auf der in **Fig. 13** rechten Seite der Anlage ist das gleiche spiegelbildlich für rechte Formhälften 60 vorgesehen. Infolge dieser Konstruktion schließt sich ein Paar aus einer linken Formhälfte 58 und einer rechten Formhälfte 60 im oberen Bereich der Anlage. Das Rohmaterial wird von oben eingefüllt. Die Endlosketten mitsamt der Formhälften wandern kontinuierlich nach unten in **Fig. 13** und durchlaufen eine Heizstation 66, danach eine Kühlstation 68. Weiter unten gehen die Formhälften auseinander, so daß der fertige Trägerkörper entnommen werden kann. Der Verfahrensablauf kann auch schrittweise sein, so daß die einzelnen Formen in der Materialeinbringstation, in der Heizstation, in der Kühlstation und in der Entnahmestation jeweils stillstehen. IN der Materialeinbringstation kann die Form zur Verdichtung des Inhalts vibriert werden.

Es versteht sich, daß bei den Ausführungsformen gemäß **Fig. 9, 10** und **11** der Formgebungsraum seitlich, d.h. jeweils vor und hinter der Zeichnungsebene, geschlossen ist. Zum Beispiel sind dort Platten angebracht, zwischen denen die Bänder 48 oder die Walzen 54 mit engem seitlichem Spiel laufen.

Wie schon mehrfach angesprochen, müssen die Kunststoffteilchen, die durch Wärmeeinwirkung miteinander verbunden werden sollen, im Formgebungsraum eine hierfür geeignete Temperatur (im Fall von Polyethylen-Teilen z.B. im Temperaturbereich von 160 bis 190°C) haben. Die bevorzugtesten Möglichkeiten, um im Formgebungsraum derartige Temperaturen zu erreichen, sind, einzeln oder in Kombination:
- Beheizung mindestens eines Teils der Begrenzungsflächen des Formgebungsraums, also z.B. Beheizen der Bänder 48 oder der Walzen 54:
- Vorwärmung der Kunststoffteilchen und/oder der Blähton-Teilchen. Die Vorwärmung der Blähton-Teilchen ist besonders effektiv, weil diese vor der Zugabe zu den Kunststoffteilchen auf höhere Temperatur vorgewärmt werden können und eine relativ hohe Wärmekapazität haben;
- sonstige Wärmezufuhr zum Inhalt des Formgebungsraums, vorzugsweise durch Hindurchblasen von warmer Luft, Erhitzung mittels Mikrowellen und dergleichen. Bei den Ausführungsformen gemäß **Fig. 9, 10** und **11** kann die letztgenannte Art der Erwärmung z.B. in Richtung rechtwinkelig zur Zeichnungsebene erfolgen.

Das bevorzugte Gewichtsverhältnis zwischen Kunststoffteilchen und feinporösen Teilchen ist 10 bis 65 %, vorzugsweise 30 bis 55 %, Kunststoffteilchen, Rest feinporöse Teilchen.

In **Fig. 14** ist veranschaulicht, wie die Kunststoffteilchen 70 und die feinporösen Teilchen 72 im fertigen Trägerkörper 14 vorliegen.

Es ist bereits angesprochen worden, daß man mittels des erfindungsgemäßen Bioreaktors 2 entweder Flüssigkeiten oder Flüssigkeiten unter Anwesenheit von Gasen behandeln kann. In beiden Fällen kann die Hauptströmung der zu behandelnden Flüssigkeit oder des zu behandelnden Gases Vorzugsweise von unten nach oben oder von oben nach unten sein. Man kann aber auch in einem Bioreaktor 2 sowohl eine Flüssigkeit als auch in Gas gleichzeitig behandeln. Flüssigkeit und Gas können dabei entweder im Gleichstrom oder im Gegenstrom strömen. Anhand von **Fig. 1** kann man sich am leichtesten vorstellen, daß zusätzlich zu dem Abwasserverteilsystem 10 ein Gasverteilsystem im unteren Bereich des Bioreaktors 2 vorgesehen ist. Abwasser und Gas strömen im Gleichstrom nach oben. Dies ist zugleich ein Beispiel für eine aerobe Abwasserbehandlung.

Auch mit den kontinuierlichen Verfahren gemäß **Fig. 9, 10** und **11** lassen sich problemlos Trägerkörper 14 mit integralen Distanzstegen 28 erzeugen, indem man z.B. Bänder 48 oder Walzen 54 mit entsprechenden Aussparungen in Längsrichtung bzw. Umfangsrichtung vorsieht.

## Patentansprüche

1. Festbett-Bioreaktor (2) zur Reinigung von Fluiden mit Hilfe von Mikroorganismen, der eine Mehrzahl von Trägerkörpern (14) für Mikroorganismen und Strömungswege (36) für das Fluid entlang der Trägerkörper (14) enthält, wobei die Trägerkörper (14) flächige Gebilde mit im Vergleich zu der Trägerkörperfläche kleiner Dicke sind, einen porösen Aufbau mit von dem Fluid durchsetzbaren und von Mikroorganismen besiedelbaren Poren haben, und mit durch Wärmeeinwirkung verbundenen Kunststoffteilchen (70) aufgebaut sind,
**dadurch gekennzeichnet,**
daß die Trägerkörper (14) mit Hilfe von Distanzstegen (28) voneinander beabstandet sind, wodurch die Strömungswege (36) zwischen den Trägerkörpern (14) definiert sind; und daß die Distanzstege (28) integral mit dem Kunststoffmaterial eines jeweils zugeordneten Trägerkörpers (14) angeformt sind.

2. Festbett-Bioreaktor nach Anspruch 1,
**dadurch gekennzeichnet,**
daß mehrere Trägerkörper (14) durch gegenseitiges Verbinden unter Einbeziehung der Distanzstege (28) zu einem Trägerkörper-Paket (38) vereinigt sind, vorzugsweise durch Klebung oder Schweißung.

3. Festbett-Bioreaktor nach Anspruch 1 oder 2,
**dadurch gekennzeichnet,**
daß die Trägerkörper (14) jeweils im wesentlichen die Gestalt einer ebenen Platte mit integralen Distanzstegen (28) haben.

4. Festbett-Bioreaktor nach mindestens einem der Ansprüche 1 bis 3,
**dadurch gekennzeichnet,**
daß die Trägerkörper (14) feinporöse Teilchen (72) enthalten.

5. Trägerkörper (14) für Festbett-Bioreaktoren (2) zur Reinigung von Fluiden mit Hilfe von Mikroorganismen, wobei der Trägerkörper (14) ein flächiges Gebilde mit im Vergleich zu der Trägerkörperfläche kleiner Dicke ist, einen porösen Aufbau mit von dem Fluid durchsetzbaren und von Mikroorganismen besiedelbaren Poren hat, und mit durch Wärmeeinwirkung verbundenen Kunststoffteilchen (70) aufgebaut ist,
**dadurch gekennzeichnet,**
daß der Trägerkörper (14) integral mit seinem Kunststoffmaterial angeformte Distanzstege (28) zur Definition des Abstands zu einem benachbarten Trägerkörper (14) aufweist.

6. Trägerkörper nach Anspruch 5,
**dadurch gekennzeichnet,**
daß er im wesentlichen die Gestalt einer ebenen Platte mit davon vorstehenden Distanzstegen (28) hat.

7. Trägerkörper nach Anspruch 5 oder 6,
**dadurch gekennzeichnet,**
daß er feinporöse Teilchen (72) enthält.

8. Verfahren zur Herstellung von zur Besiedlung mit Mikroorganismen geeigneten Trägerkörpern (14) porösen Aufbaus, wobei das Verfahren folgende Schritte aufweist;
(a) Bereitstellen von Kunststoffteilchen (70);
(b) Einbringen der Kunststoffteilchen (70) in einen Formgebungsraum (52); und
(c) Wärmezufuhr zu den im Formgebungsraum (52) enthaltenen Kunststoffteilchen (70), so daß sich die Kunststoffteilchen (70) unter Ausbildung des porösen Trägerkörperaufbaus miteinander verbinden,
**dadurch gekennzeichnet,**
(d) daß der Inhalt des Formgebungsraums (52) durch Bewegung bewegbarer Formgebungsraum-Begrenzungen (48;54) in Richtung zu einem Abgabeende des Formgebungsraums (52) mitgenommen wird und im Verlauf der Mitnahmebewegung durch Wärmezufuhr zu dem porösen Trägerkörperaufbau verbunden wird.

9. Verfahren nach Anspruch 8,
**dadurch gekennzeichnet,**
daß der Formgebungsraum (52) auf zwei gegenüberliegenden Seiten durch endlose, umlaufend bewegbare Bänder (48) begrenzt ist.

10. Verfahren nach Anspruch 8,
**dadurch gekennzeichnet,**
daß der Formgebungsraum (52) auf zwei gegenüberliegenden Seiten durch Walzen (54) begrenzt ist.

11. Verfahren nach mindestens einem der Ansprüche 8 bis 10,
**dadurch gekennzeichnet,**
daß die Kunststoffteilchen (70) kontinuierlich in den Formgebungsraum (52) eingebracht werden.

12. Verfahren nach mindestens einem der Ansprüche 8 bis 11,
**dadurch gekennzeichnet,**
daß die Kunststoffteilchen (70) vor dem Einbringen in den Formgebungsraum (52) vorgewärmt werden.

13. Verfahren zur Herstellung von zur Besiedlung mit Mikroorganismen geeigneten Trägerkörpern (14) porösen Aufbaus, wobei das Verfahren folgende Schritte aufweist:
(a) Bereitstellen von Kunststoffteilchen (70);
(b) Einbringen der Kunststoffteilchen (70) in einen Formgebungsraum (58,60); und
(c) Wärmezufuhr zu den im Formgebungsraum (58,60) enthaltenen Kunststoffteilchen (70), so daß sich die Kunststoffteilchen (70) unter Ausbildung des porösen Trägerkörperaufbaus miteinander verbinden,
**dadurch gekennzeichnet,**
(d) daß der jeweilige Formgebungsraum von einer ersten und einer zweiten Formhälfte (58,60) begrenzt ist;
(e) daß eine Reihe der ersten Formhälften (58) an einem ersten Förderer und eine Reihe der zweiten Formhälften (60) an einem zweiten Förderer befestigt ist;
(f) daß die Formhälften (58,60) in geschlossenem Zustand durch eine Station zum Einbringen der Kunststoffteilchen (70) und durch eine Station (66) zur Wärmezufuhr zu den beinhalteten Kunststoffteilchen (70) bewegbar sind; und
(g) daß die Formhälften (58,60) aufgrund der Ausbildung des ersten und des zweiten Förderers selbsttätig geöffnet und geschlossen werden.

14. Verfahren nach mindestens einem der Ansprüche 8 bis 13,
**dadurch gekennzeichnet,**
daß der Formgebungsraum (52;58,60) so gestaltet ist, daß Trägerkörper (14) mit einstückig angeformten Distanzstegen (28) hergestellt werden.

15. Verfahren nach Anspruch 14,
**dadurch gekennzeichnet,**
daß Begrerzungen des Formgebungsraums (52;58,60) auswechselbar sind oder bedarfsweise mit Materialeinlagen ausrüstbar sind, um Trägerkörper (14) mit Distanzstegen (28) unterschiedlicher Höhe herstellen zu können.

16. Verfahren nach mindestens einem der Ansprüche 8 bis 15,
**dadurch gekennzeichnet,**
daß den Kunststoffteilchen (70) feinporöse Teilchen (72) zugesetzt werden.

17. Verfahren nach mindestens einem der Ansprüche 8 bis 16,
**dadurch gekennzeichnet,**
daß den im Formgebungsraum (52;58,60) enthaltenen Kunststoffteilchen (70) durch Erwärmen von Begrenzungsflächen des Formgebungsraums (52;58.60) und/oder durch Mikrowellen und/oder durch Wärmestrahlung und/oder durch die Zugabe vorgewärmter, feinporöser Teilchen (72) Wärme zugeführt wird.

## Claims

1. A fixed-bed bioreactor (2) for purifying fluids with the aid of microorganisms, containing a plurality of carrier bodies (14) for microorganisms and flow paths (36) for the fluid along the carrier bodies (14), with said carrier bodies (14) being sheet-like structures with a small thickness in comparison with the carrier body surface and having a porous structure with pores adapted to be penetrated by the fluid and to have microorganisms attach thereto, and being composed with plastics particles (70) bonded together by the application of heat,
characterized in that the carrier bodies (14) are spaced apart with the aid of spacing bars (28), defining the flow paths (36) between the carrier bodies (14); and in that the spacing bars (28) are formed integrally with the plastics material of a respectively associated carrier body (14).

2. A fixed-bed bioreactor according to claim 1,
characterized in that a plurality of carrier bodies (14) is united to form a carrier body package (38) by mutual connection thereof with inclusion of the spacing bars (38), preferably by adhesive bonding or by welding.

3. A fixed-bed bioreactor according to claim 1 or 2,
characterized in that the carrier bodies (14) each have substantially the shape of a planar plate with integral spacing bars (28).

4. A fixed-bed bioreactor according to at least one of claims 1 to 3,
characterized in that the carrier bodies (14) contain fine-porosity particles (72).

5. A carrier body (14) for fixed-bed bioreactors (2) for purifying fluids with the aid of microorganisms, said carrier body (14) being a sheet-like structure with a small thickness in comparison with the carrier body surface and having a porous structure with pores adapted to be penetrated by the fluid and to have microorganisms attach thereto, and being composed with plastics particles (70) bonded together by the application of heat,
characterized in that the carrier body (14) has spacing bars (28) formed integrally with its plastics material for defining the distance to an adjacent carrier body (14).

6. A carrier body according to claim 5,
characterized in that it has substantially the shape of a planar plate with spacing bars (28) projecting therefrom.

7. A carrier body according to claim 5 or 6,
characterized in that it contains fine-porosity particles (72).

8. A process for producing carrier bodies (14) of porous structure adapted to have microorganisms attach thereto, said process comprising the following steps:
(a) providing plastics particles (70);
(b) introducing the plastics particles (70) into a moulding space (52); and
(c) supplying heat to the plastics particles (70) contained in the moulding space (52) such that the plastics particles (70) are bonded together forming the porous carrier body structure,
characterized in
(d) that the contents of the moulding space (52), by movement of movable moulding space confining means (48; 54), are entrained in the direction towards a discharge end of the moulding space (52) and in the course of the entraining movement are bonded together to form the porous carrier body structure by supply of heat.

9. A process according to claim 8,
characterized in that the moulding space (52) is confined on two opposed sides by endless revolving belts (48).

10. A process according to claim 8,
characterized in that the moulding space (52) is confined on two opposed sides by rollers (54).

11. A process according to at least one of claims 8 to 10,
characterized in that the plastics particles (70) are introduced continuously into the moulding space (52).

12. A process according to at least one of claims 8 to 11,
characterized in that the plastics particles (70) are preheated prior to introduction into the moulding space (52).

13. A process for producing carrier bodies (14) of porous structure adapted to have microorganisms attach thereto, said process comprising the following steps:
(a) providing plastics particles (70);
(b) introducing the plastics particles (70) into a moulding space (58, 60); and
(c) supplying heat to the plastics particles (70) contained in the moulding space (58, 60) such that the plastics particles (70) are bonded together forming the porous carrier body structure,
characterized in
(d) that the particular moulding space is confined by a first and a second mould half (58, 60);
(e) that a number of the first mould halves (58) is mounted on a first conveyor and a number of the second mould halves (60) is mounted on a second conveyor;
(f) that the mould halves (58, 60) in the closed condition thereof are adapted to be moved through a station for introduction of the plastics particles (70) and through a station (66) for the application of heat to the introduced plastics particles (70); and
(g) that the mould halves (58, 60), due to the design of said first and second conveyors, are closed and opened automatically.

14. A process according to at least one of claims 8 to 13,
characterized in that the moulding space (52; 58, 60) is designed such that carrier bodies (14) with spacing bars (28) integrally formed thereon are produced.

15. A process according to claim 14,
characterized in that the confining means of the moulding space (52; 58, 60) can be exchanged or, if necessary, can be equipped with material inserts so as to be able to produce carrier bodies (14) having spacing bars (28) of different height.

16. A process according to at least one of claims 8 to 15,
characterized in that fine-porosity particles (72) are added to the plastics particles (70).

17. A process according to at least one of claims 8 to 16,
characterized in that heat is supplied to the plastics particles (70) contained in the moulding space (52; 58, 60) by heating confinement areas of the moulding space (52; 58, 60) and/or microwaves and/or heat radiation and/or addition of preheated fine-porosity particles (72).

## Revendications

1. Bioréacteur à lit fixe (2) pour l'épuration de fluides à l'aide de micro-organismes qui contient une pluralité de corps de support (14) pour les micro-organismes et des trajets d'écoulement (36) pour le fluide le long des corps de support (14), les corps de support étant des produits minces présentant une faible épaisseur par rapport à la surface des corps de support, ayant une constitution poreuse avec des pores pouvant être traversés par le fluide et pouvant être colonisés par les micro-organismes et étant formés de particules de matériau synthétique liées sous l'effet de la chaleur,
caractérisé en ce que
les corps de support (14) sont écartés les uns des autres à l'aide d'entretoises (28), les trajets d'écoulement (36) étant ainsi définis entre les corps de support (14);
et en ce que les entretoises (28) viennent de matière avec le matériau synthétique d'un corps de support (14) respectivement associé.

2. Bioréacteur à lit fixe selon la revendication 1,
caractérisé en ce que
plusieurs corps de support (14) sont réunis en un paquet de corps de support (38) par liaison réciproque, de préférence par collage ou par soudage, avec intégration des entretoises (28).

3. Bioréacteur à lit fixe selon la revendication 1 ou 2,
caractérisé en ce que
les corps de support (14) ont chacun la forme générale d'une plaque plane comprenant des entretoises (28) venues de matière.

4. Bioréacteur à lit fixe selon l'une au moins des revendications 1 à 3,
caractérisé en ce que
les corps de support (14) contiennent des particules (72) finement poreuses.

5. Corps de support (14) pour réacteurs à lit fixe (2) pour l'épuration de fluides à l'aide de micro-organismes, le corps de support (14) étant un produit plat de faible épaisseur par rapport à la surface du corps de support, possédant une constitution poreuse comprenant des pores pouvant être traversés par le fluide et pouvant être colonisés par des micro-organismes et étant formé de particules de matériau synthétique liées sous l'effet de la chaleur,
caractérisé en ce que
le corps de support (14) présente des entretoises (28) venant de matière avec son matériau synthétique pour définir l'écartement avec un corps de support (14) voisin.

6. Corps de support selon la revendication 5,
caractérisé en ce
qu'il a généralement la forme d'une plaque plane dont font saillie des entretoises (28).

7. Corps de support selon la revendication 5 ou 6,
caractérisé en ce
qu'il contient des particules finement poreuses (72).

8. Procédé de fabrication de corps de support (14) de constitution poreuse appropriés pour la colonisation par des micro-organismes, comprenant les étapes suivantes :
(a) mise à disposition de particules de matériau synthétique (70);
(b) introduction des particules de matériau synthétique (70) dans une cavité de moulage (52); et
(c) fourniture de chaleur aux particules de matériau synthétique (70) contenues dans la cavité de moulage (52) de façon que les particules de matériau synthétique (70) se Tient entre elles en réalisant la constitution poreuse du corps de support,
caractérisé en ce que
(d) le contenu de la cavité de moulage (52) est entraîné par déplacement de délimitations (48; 54) mobiles de cavité de moulage vers une extrémité de délivrance de la cavité de moulage (52) et en ce qu'au cours du déplacement par entraînement il se transforme par liaison par fourniture de chaleur en la constitution poreuse du corps de support.

9. Procédé selon la revendication 8,
caractérisé en ce que
la cavité de moulage (52) est délimitée sur deux côtés opposés par des bandes sans fin mobiles circulant.

10. Procédé selon la revendication 8,
caractérisé en ce que
la cavité de moulage (52) est délimitée sur deux côtés opposés par des rouleaux (54).

11. Procédé selon l'une au moins des revendications 8 à 10,
caractérisé en ce que
les particules de matériau synthétique (70) sont introduites en continu dans la cavité de moulage (52).

12. Procédé selon l'une au moins des revendications 8 à 11,
caractérisé en ce que
les particules de matériau synthétique (70) sont préchauffées avant d'être introduites dans la cavité de moulage (52).

13. Procédé de fabrication de corps de support (14) de constitution poreuse appropriés pour être colonisés par des micro-organismes, comprenant les étapes suivantes :
(a) mise à disposition de particules de matériau synthétique (70);
(b) introduction des particules de matériau synthétique (70) dans une cavité de moulage (58, 60); et
(c) fourniture de chaleur aux particules de matériau synthétique (70) contenues dans la cavité de moulage (58, 60) de façon que les particules de matériau synthétique (70) se lient entre elles en réalisant la constitution poreuse du corps de support,
caractérisé en ce que
(d) chaque cavité de moulage respective est délimitée par une première et une seconde moitiés de moule (58, 60);
(e) une série des premières moitiés de moule (58) sont fixées à un premier convoyeur et une série des secondes moitiés de moule (60) sont fixées à un second convoyeur;
(f) les moitiés de moule (58, 60) peuvent être déplacées à l'état fermé à travers un poste d'introduction des particules de matériau synthétique (70) et à travers un poste (66) de fourniture de chaleur aux particules de matériau synthétique (70) contenues; et
(g) les moitiés de moule (58, 60) sont ouvertes et fermées automatiquement en raison de la configuration du second convoyeur.

14. Procédé selon l'une au moins des revendications 8 à 13,
caractérisé en ce que
la cavité de moulage (52; 58, 60) est conformée de façon à fabriquer des corps de support (70) comportant des entretoises (28) venues de matière avec eux.

15. Procédé selon la revendication 14,
caractérisé en ce que
des délimitations de la cavité de moulage (52; 58, 60) sont interchangeables ou en cas de besoin peuvent être équipées de garnitures de matériau pour pouvoir fabriquer des corps de support (14) pourvus d'entretoises (28) de différentes hauteurs.

16. Procédé selon l'une au moins des revendications 8 à 15,
caractérisé en ce que
des particules finement poreuses (72) sont ajoutées aux particules de matériau synthétique (70).

17. Procédé selon l'une au moins des revendications 8 à 16,
caractérisé en ce que
de la chaleur est fournie aux particules de matériau synthétique (70) contenues dans la cavité de moulage (52; 58, 60) en chauffant des surfaces de délimitation de la cavité de moulage (52; 58, 60) et/ou par micro-ondes et/ou par rayonnement thermique et/ou en ajoutant des particules finement poreuses (72) préchauffées.
